# EUROPEAN PATENT SPECIFICATION

(11) **EP 3 103 487 B1**
(45) Date of publication and mention of the grant of the patent: **11.03.2020**
(21) Application number: 16275068.1
(22) Date of filing: 29.04.2016
(51) Int. Cl.: A61L 31/02, A61L 31/16

(54) **BIOACTIVE MATERIAL COATED MEDICAL DEVICE**
MIT BIOAKTIVEM MATERIAL BESCHICHTETE MEDIZINISCHE VORRICHTUNG
DISPOSITIF MÉDICAL REVÊTU D'UN MATÉRIAU BIOACTIF

(30) Priority: 09.06.2015 GB 201510015; 09.06.2015 US 201562172972 P
(43) Date of publication of application: 14.12.2016
(73) Proprietor: Cook Medical Technologies LLC, Bloomington, IN 47404 (US)
(72) Inventor: MURRAY, David, Limerick (IE); NEILAN, John, Limerick (IE)
(74) Representative: Williams Powell

(56) References cited:
- US-A1- 2005 119 723
- US-A1- 2009 171 453
- US-A1- 2014 178 563

## Description

### TECHNICAL FIELD

The present invention relates to a medical device having a bioactive material coated thereon and a method for manufacturing said device.

### BACKGROUND

Conditions and diseases of the body may be treated by implanting a medical device within the patient. Such medical devices may be coated with a bioactive material suitable for treating the condition or disease, or for preventing further conditions or diseases from arising as a result of the surgery. For example, stenosed blood vessels may be treated by inserting a stent into the vessel so as to open the vessel and allow proper blood flow through it. However, after the procedure the presence of the stent in the vessel may cause fibrosis of the surrounding tissue and development of thrombi, which together may cause further stenosis or occlusion of the vessel. In order to reduce the chances of such restenosis occurring, the stent may be coated with a drug such as Paclitaxel to inhibit cell proliferation around the implanted device.

Other conditions and diseases are treatable with stents, catheters, cannulae and other medical devices inserted into the esophagus, trachea, colon, biliary tract, urinary tract and other locations in the body, or with orthopedic devices, implants, or replacements. Any of these devices may be coated with bioactive materials to be delivered in the vicinity of the implantable device. Polymer based carrier materials have been used to adhere drugs to stents for delivery into a patient, however the polymer carriers can have adverse side effects which are undesirable for the patient.

US-2014/0178563 discloses providing a stent with a roughened or textured surface for increasing the adhesion of a bioactive material to the stent. US-2005/0119723 discloses an implantable medical device with a porous surface with a composite material located within the pores that includes a bioerodable material in combination with a bioactive agent. The composite material is designed to erode upon exposure to the body of a patient. US-2009/0171453 discloses drug coated stents in which the luminal surface of the stent is treated with a protein-adherence deterring coating.

### SUMMARY

The present invention seeks to provide an improved implantable medical device having a bioactive material coated thereon, and an improved method for manufacturing said device.

According to an aspect of the invention there is provided an implantable medical device as specified in claim 1 and a method of making an implantable medical device according to claim 12.

The implantable medical device includes at least one contact surface having a bioactive material coated directly thereon is provided, the at least one contact surface having a defined total surface energy density, wherein the bioactive material has a defined total surface energy density, wherein the total surface energy densities of the contact surface and the bioactive material are preferably substantially matched.

The surface energies of the at least one contact surface and of the bioactive material each comprise a polar component and a dispersive (otherwise known as non-polar) component. The polar components of the surface energies are substantially matched and the dispersive (non-polar) components of the surface energies are substantially matched.

The surface energies and components thereof may be measured using the Owens, Wendt, Rabel and Kaelble method (OWRK method).

Where the medical device comprises nitinol, and the bioactive material is predominantly non-polar, the non-polar component of the surface energy of the at least one contact surface may be greater than 40%, and preferably substantially 50% of the surface energy of the at least one contact surface.

There is also described a method of making an implantable medical device including a contact surface having a bioactive material coated directly thereon, the contact surface having a defined surface energy density and the bioactive material having a defined surface energy density; the method comprising the steps of:
substantially matching the total surface energy densities of the contact surface and the bioactive material.

Where the surface energies of the at least one contact surface and of the bioactive material each comprise a polar component and a dispersive (otherwise known as non-polar) component the method may comprise the steps of:
substantially matching the polar components of the surface energies; and
substantially matching the dispersive (non-polar) components of the surface energies.

The medical device may include a structure comprising a base material. The at least one contact surface may be a surface of the base material. The contact surface may comprise at least one of non-polar and polar components. The polar and non-polar components of the at least one contact surface may be present in a given proportion or ratio. The polar components may provide the polar component of the surface energy, while the non-polar components may provide the non-polar component of the surface energy.

The bioactive material may be predominantly polar or non-polar. The polar and non-polar components of the bioactive material may be present in a given proportion or ratio. The at least one contact surface may be a modified surface. The surface may have been modified to alter the ratio or proportion of polar and non-polar components in the direction of, or closer to the ratio or proportion of polar and non-polar components in the bioactive material. The surface may have been modified to increase a proportion of non-polar components where the bioactive material is predominantly non-polar and to decrease the proportion of non-polar components where the bioactive material is predominantly polar.

Also described is an implantable medical device including a structure comprising a base material, the structure including at least one contact surface of the base material, the contact surface comprising at least one of non-polar and polar components; and a bioactive material coating disposed directly on the at least one contact surface, the bioactive material being predominantly polar or non-polar; the at least one contact surface being modified to alter the proportion of polar and non-polar components in the direction of the proportion of polar and non-polar components in the bioactive material.

A surface energy of the at least one contact surface may be above about 40 Dynes/cm (40mN/m).

Altering the proportion of polar and non-polar components in the direction of the proportion of polar and non-polar components in the bioactive material can be increasing a proportion of non-polar components where the bioactive material is predominantly non-polar and decreasing the proportion of non-polar components where the bioactive material is predominantly polar.

The polar and non-polar components at the contact surface may be formed from the bulk material, for example, such as the formation of a metal oxide layer at the surface of a metal material in air, or may comprise the bulk material. However, such surfaces may not be suitable for, or effective at binding a bioactive material to said surface. Providing a modified surface enables improved binding of the bioactive material to the surface without the need for an additional component such as a polymer layer to hold the bioactive material in place.

The surface energy of the at least one contact surface may be between about 40 and 60 Dynes/cm (40-60 mN/m), between about 40 and 50 Dynes/cm (40-50 mN/m), or between about 50 and 60 Dynes/cm (50-60 mN/m).

Providing a surface having a higher surface energy enables the bioactive material to better adhere to said surface. The inventors have found that providing a contact surface having a surface energy in these ranges enables good adhesion of the bioactive material to the surface, whilst still enabling the drug to be released from the surface and pass into the body. In particular, where the bioactive material is non-polar, a surface energy of between about 40 and 50 Dynes/cm (40-50 mN/m) can allow an effective amount of the bioactive material to be released from the surface, after delivery in the patient, into the body.

The surface energy of the at least one contact surface may be substantially matched to a surface energy of the bioactive material.

Substantially matching the surface energies or components may be matching to within +/- 15 Dynes/cm (15 mN/m). Preferably, matching is to within +/- 10 Dynes/cm (10 mN/m), and more preferably to within +/- 5 Dynes/cm (5 mN/m). Substantially matching the surface energies or components may be matching to within +/- 2 to 9 %.

Where the surface energy of the contact surface is 40 Dynes/cm (40 mN/m), substantially matching the surface energy of the bioactive material may be matching to within +/- 2 %, +/- 4 %, or +/- 6 % of the surface energy of the contact surface. Where the surface energy of the contact surface is 50 Dynes/cm (50 mN/m), substantially matching the surface energy of the bioactive material may be matching to within +/- 2.5 %, +/- 5 %, +/- 7.5 % of the surface energy of the contact surface. Where the surface energy of the contact surface is 60 Dynes/cm (60 mN/m), substantially matching the surface energy of the bioactive material may be matching to within +/- 3 %, +/- 6 %, +/- 9 % of the surface energy of the contact surface.

Matching the surface energies of the bioactive material and the contact surface can allow optimised bonding and elution of the material from the surface when in situ.

The at least one contact surface may be selectively etched to remove at least some of one of the polar and non-polar components at the at least one contact surface.

Etching is a relatively harsh technique which can strip layers off the surface of the device. Etching may allow new components to form from the base material at the surface. Etching can selectively remove specific polar or non-polar components from the surface. By tailoring the etching process, including the solution and time for etching etc, to remove selected amounts of specific compounds at the contact surface, the contact surface can be modified so as to optimise binding to a given bioactive material. The etching process may involve immersing the contact surface in an acid, or alkali solution.

The at least one contact surface may be selectively polished to remove at least some of one of the polar and non-polar components at the at least one contact surface. Selective polishing can provide the same advantages as selective etching. Selective polishing may include electro-polishing.

The at least one contact surface may be at least partially passivated or ionised to alter the proportion of polar to non-polar components at the at least one contact surface. Passivating has the advantage of preventing further reaction at the surface, thereby rendering the surface inert. Ionising the surface may include bombarding the surface with an ion beam of an inert gas, such as argon, for example.

Ion beam penetration may be used to alter the proportion of polar to non-polar components at the at least one contact surface. The at least one contact surface may be impregnated with a material by ion bean penetration. Such a process may therefore modify the at least one contact surface.

The base material may be selectively doped so as to modify the at least one contact surface, said doping altering the proportion of polar to non-polar components at the at least one contact surface. The doping element may be selected so as to modify the surface formed on the base material to more favourably bind to a particular bioactive material.

Any combination of these techniques may be used to modify the contact surface of the device.

The medical device may comprise an alloy or a polymer. The base material may comprise a nickel titanium alloy. The at least one contact surface may comprise nickel, in the form of nickel ions and also in nickel containing compounds. Some of the nickel containing compounds may be polar. The at least one contact surface may be chemically treated to reduce the amount of nickel at said surface. In particular, the at least one contact surface may comprise between 0 and 5 at%, preferably between 0 to 3 at%, and more preferably between 1.2 at% and 2.5 at% nickel. The at least one contact surface may comprise between about 1 and 2 at% nickel. The at least one contact surface may comprise no less than 1 at % nickel. The at least one contact surface may comprise and no more than 2, 2.5, 3, or 5 at % nickel. The percentage of nickel present at the surface may be measured by X-ray Photoelectron Spectroscopy (XPS). Where the bioactive material is non-polar, such a reduction in nickel and polar nickel containing compounds can facilitate optimised adherence of the bioactive material to the contact surface.

The medical device may be a stent. The bioactive material may be Paclitaxel. The Paclitaxel may be at least 80% amorphous.

The at least one contact surface may have a thickness of between 45 and 65 x 10⁻¹⁰m.

The structure may include at least one second surface, the at least one second surface having a proportion of polar and non-polar components different from the proportion of polar and non-polar components of the at least one contact surface.

The at least one second surface may be coated with a second bioactive material for treating the patient. Taking the example of a luminal medical device for delivery into a vessel, the at least one first surface may be on the outer luminal surface of the device, and the at least one second surface on the inner luminal surface of the device. Such an arrangement can allow delivery of the first bioactive material into the lumen wall, and of the second bioactive material into the fluid flowing through the vessel.

According to another aspect of the present invention a method is provided for making an implantable medical device including a structure comprising a base material, the structure including at least one contact surface of the base material, the contact surface comprising non-polar and polar components; and a bioactive material coating disposed directly on the at least one contact surface, the bioactive material being predominantly polar or non-polar; the method including the steps of:
modifying the at least one contact surface by altering the proportion of polar and non-polar components to be closer to the proportion of polar and non-polar components in the bioactive material; matching the polar component of the surface energy of the at least one contact surface (14) to within +/- 15 mN/m of the polar component of the surface energy of the bioactive material and matching the non-polar component of the surface energy of the at least one contact surface to within +/- 15 mN/m of the non-polar component of the surface energy of the bioactive material; and
applying the bioactive material directly to the at least one contact surface;
wherein the surface energy of the at least one contact surface is above 40 Dynes/cm (40mN/m).

The step of treating the at least one contact surface prior to application of the bioactive material may limit the surface energy of the at least one contact surface to between 40 and 60 mN/m.

The method may include the step of substantially matching the surface energy of the at least one contact surface to a surface energy of the bioactive material.

The modification step may include chemically modifying at least a part of the at least one contact surface.

The modification step may include at least one of selectively etching, selectively polishing, passivating or ionising the at least one contact surface to remove at least some of one of the polar and non-polar components at the at least one contact surface. Selective polishing may include electropolishing.

The modification step may include selectively doping the base material so as to alter the proportion of polar to non-polar components at the at least one contact surface.

Wherein the at least one contact surface comprises nickel and nickel containing compounds, the step of modification may include chemically treating the at least one surface to reduce the amount of nickel at said surface.

The step of modification may include chemically treating the at least one surface to reduce the amount of nickel and nickel containing compounds at said surface such that the surface comprises no more than 1-2% nickel.

The modification step may modify the base material of the contact surface to a thickness of between 45 and 65 x 10⁻¹⁰m.

The method may include the step of forming at least one second surface, the at least one second surface having a different proportion of polar to non-polar components than the proportion of polar to non-polar components of the at least one contact surface.

Also described is a method of making an implantable medical device including at least one contact surface having a bioactive material coated directly thereon, the at least one contact surface having a defined surface energy density and the bioactive material having a defined surface energy density, the method comprising the steps of:
substantially matching the total surface energy densities of the at least one contact surface and the bioactive material; and
coating the bioactive material directly on the at least one contact surface.

The surface energies of the at least one contact surface and of the bioactive material each comprises a polar component and a non-polar component, and the method comprises the steps of:
matching the polar components of the surface energies of the at least one contact surface and the bioactive material to within +/- 15 mN/m of another; and
matching the non-polar components of the surface energies of the at least one contact surface and the bioactive material to within +/- 15 mN/m of each another.

### DESCRIPTION

Embodiments of the present invention are described below, by way of example only, with reference to the accompanying drawings, in which:
Figure 1 shows a cross-sectional view of a medical device coated with a bioactive material;
Figure 2 is a schematic view of a stent coated with a first bioactive material; and
Figure 3 is a flow diagram showing a method for making an implantable medical device according to an embodiment of the invention.

The components in the drawings are not necessarily to scale, emphasis instead being placed upon illustrating the principles of the teachings herein.

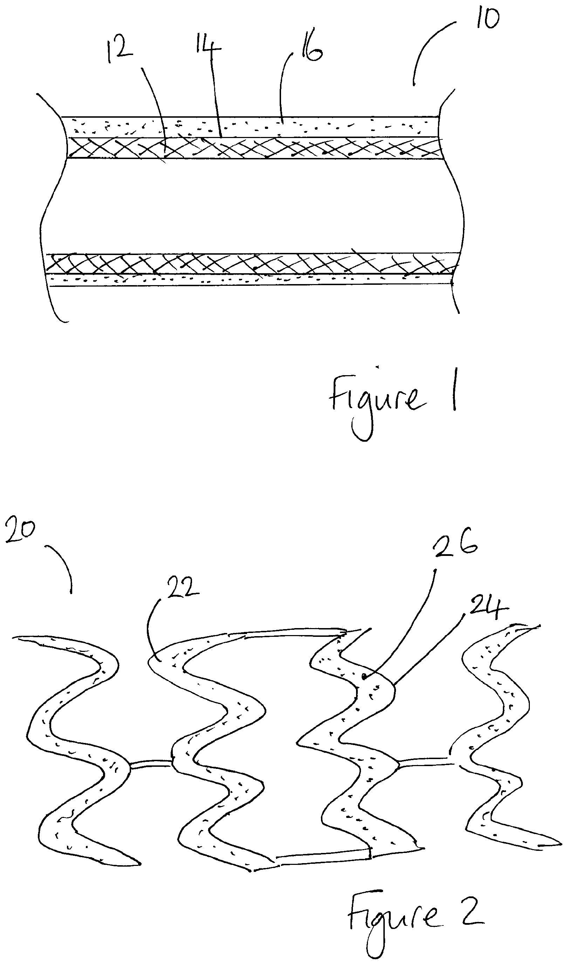

Figure 1 shows a cross-sectional view of a medical device 10. The medical device 10 comprises a base material 12 having a first contact surface 14 which is coated with a bioactive material 16. The bioactive material 16, which can be classed as more polar or more non-polar in nature, may be any drug, medicament or material, or combination thereof which can be coated onto a contact surface. The bioactive material preferably forms a layer on the contact surface, the layer containing about 3 µg per mm² of the surface. Approximately 95% of this dose may be released within 24 hours of deployment of the device. The remainder of the 3 µg/mm² may be released over the coming days or months of implantation.

Where the medical device is a stent, the contact surface may be the abluminal surface of the stent. The amount of bioactive material on the surface can be determined by High Performance Liquid Chromatography (HPLC).

The contact surface 14 is an outer layer of the base material of the device 10; the contact surface may have a thickness or depth of, for example, several atoms or molecules. The surface may have a thickness in the region of several nanometres. The chemical composition of the contact surface largely determines how the device 10 interacts with materials placed on that device. Components of the contact surface are classified as polar or non-polar. By polar it is meant that a dipole moment exists between atoms in a component at the surface. By non-polar it is meant that no dipole moment exists between the atoms.

The surface energy of the bioactive material will depend on the polar or non-polar nature of the material. Where the material is a powder, the surface energy may be determined by a theoretical method such as the Parachor method, for example.

The surface energy density of a solid surface can be measured by various known methods. One method, known as the Owens, Wendt, Rabel and Kaelble (OWRK) method, involves measuring the contact angle of the surface with several known liquids, such as water, diiodomethane and 1,5-pentanediol, then using the OWRK equations to calculate the surface energy. This method breaks the surface energy density of the contact surface down into polar and non-polar components, each component representing the energy contributed by the respective polar and non-polar components at that surface. In a preferred embodiment, the at least one contact surface of the device has a surface energy of at least 40 Dynes/cm (40mN/m).

Preferably, the total surface energy density of the contact surface and the total surface energy density of the bioactive material are substantially matched to one another. Even more preferably, the polar components of the surface energy of the contact surface and the polar components of the surface energy density of the bioactive material are substantially matched to one another, and the non-polar components of the surface energy of the contact surface and the non-polar components of the surface energy density of the bioactive material are substantially matched to one another. The at least one contact surface may be modified such that the total surface energy of the contact surface substantially matches that of the bioactive material.

Once the energies have been matched, the bioactive material may be coated directly on the at least one contact surface.

According to aspects of the present invention the surface of the device can be modified by adjusting the proportions of polar and non-polar components at the surface to optimise bonding of the surface with the bioactive material selected for the coating. When considering the modification to be made, regard is had not just to the strength of the bond the bioactive material can make with the contact surface of the device but also to the ability of the surface to release the bioactive material once the device is in position inside the patient. If the bioactive material is adhered to the medical device excessively strongly, it cannot be released from the device into the patient and thus cannot treat the condition or disease it is provided to treat. In an embodiment an upper limit of approximately 60 Dynes/cm (60 mN/m) is preferred.

Where the bioactive material is non-polar, the inventors have found that adherence of the bioactive material to a surface can be improved, even optimised, by increasing the proportion of non-polar components at the contact surface. This may be done, for example, by selectively removing polar components from the surface. For polar bioactive materials, the inventors have found that increasing the proportion of polar components at the surface improves bonding of said bioactive material to said surface.

In general, a polar surface component has a higher energy than a non-polar surface as a dipole moment increases the Gibbs free energy of the surface (surface energy). As such, a given percentage of polar components at the surface will contribute a greater percentage of the total surface energy. The inventors have found that for non-polar bioactive materials, bonding to a nitinol surface is optimised by increasing the proportion of non-polar components at the surface such that approximately 50% of the total surface energy is provided by them.

Figure 2 is a schematic view of a stent 20 formed from nitinol 22, the stent having a contact surface 24 coated with Paclitaxel drug 26. The stent shown is for use in the superficial femoral artery, although the skilled person will understand that the invention can be carried out in a similar way for other types of stents.

Nitinol is an alloy of nickel and titanium, where the two elements are present in roughly equal atomic percentages, for example nitinol 55 and nitinol 60. Nitinol a biocompatible superelastic shape-memory material which is used in stents for its ability to be delivered into a patient in a collapsed configuration and to self-expand when released inside the body.

During manufacture the stent is exposed to the atmosphere, heated, and generally handled. Metal atoms at the surface spontaneously oxidise, with formation of titanium dioxide being particularly energetically favourable. X-ray Photoelectron Spectroscopy (XPS) has been used to characterise the contact surface 24 of a nitinol stent 20. The surface was found to comprise, amongst other components, titanium dioxide, titanium, nickel, nickel oxides and hydroxides. Oxides of carbon, calcium and silicon were also observed using the XPS method. Table 1 shows the components and their relative amounts at the contact surface, as determined by X-ray Photoelectron Spectroscopy (XPS).

**Table 1 - XPS Results**

| **O** | **C** | **Ti** | **Ni** | **N** | **Ca** | **Si** |
|---|---|---|---|---|---|---|
| 51.74 % | 31.40 % | 11.51 % | 0.46 % | 0.21 % | 1.07 % | 3.60 % |

The contact surface of the nitinol stent was shown to have a thickness of between 45 and 65 x 10⁻¹⁰m using Auger Electron Spectroscopy (AES).

Of the components at the surface, titanium dioxide, and nickel monoxide are considered non-polar, whereas titanium, nickel, and nickel hydroxide are considered polar. Table 2 shows the polar and non-polar components of the surface energy, together with the total surface energies for nitinol stents having different surface compositions.

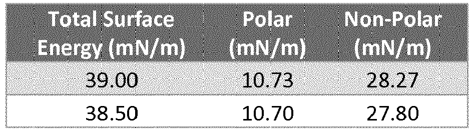

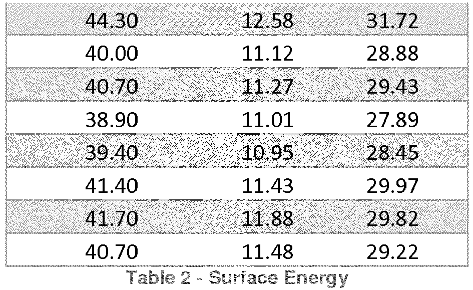

The surface energy of Paclitaxel may be determined theoretically by the Parachor method, for example, or via experimental techniques. The surface energy of Paclitaxel has been shown to be between 40 and 60 Dynes/cm (40-60 Nm/m).

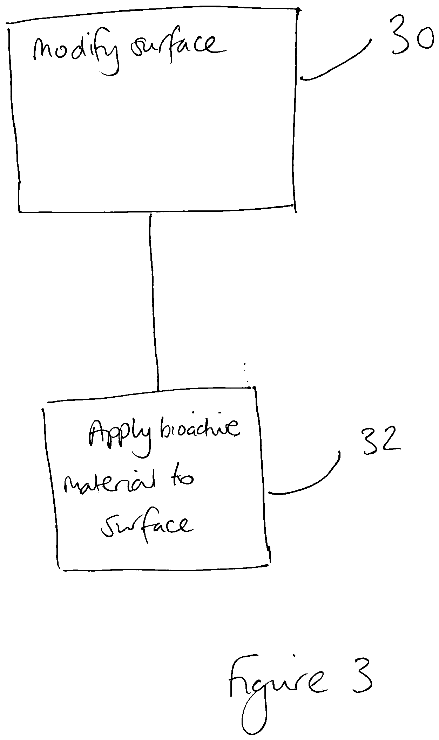

Figure 3 is a flow diagram showing the steps of a method for making an implantable medical device as shown in figure 2. The method includes the steps of: modifying the at least one contact surface of the device to increase a proportion of non-polar components, as the bioactive material is predominantly non-polar 30; and applying the bioactive material directly to the at least one contact surface 32.

In the step of modifying the at least one contact surface of the device, the surface is modified by selectively removing nickel and nickel hydroxide from the surface. This can be achieved by etching the surface using a hydrofluoric acid solution for a number of minutes. This first step may remove at least some of the oxides present on the surface of the stent following the manufacturing process. The method may include further steps of etching the surface using a mixture of hydrofluoric acid, nitric acid, and methanol, or etching the surface using sulfuric acid and methanol solution, for example. Each etching step may be carried out for a number of minutes. The later etching steps may allow controlled growth of a titanium dioxide layer on the surface of the base nitinol material.

In another embodiment a nitinol contact surface may be polished using a solution of Hydrofluoric acid and methanol. This method may be carried out at, for example, -30°C.

Preferably, nickel is removed from the surface until only 1 at% to 2 at% nickel remains. The amount of nickel can be measured by X-ray Photoelectron Spectroscopy (XPS). Generally, a small amount of nickel on the surface of the stent is desirable to prevent corrosion of the surface.

In another embodiment a stainless steel contact surface may be passivated in a solution of, for example nitric acid.

In the step of applying the bioactive material directly to the at least one contact surface, the Paclitaxel is deposited on the contact surface of the stent by spraying a solution of the Paclitaxel dissolved in ethanol, acetone or chloroform, for example, onto the surface using an airbrush type applicator. Such a spray has been found to provide a coating of reliable uniformity and provides the greatest control over the amount of bioactive material deposited on the surface. Multiple sprays may be required to provide the desired coating. Surfaces which are not to be coated may be masked prior to spraying, and the mask subsequently removed. Preferably approximately 2.5 to 3 micrograms per square millimetre is deposited on the surface of the stent.

Paclitaxel 26 is an anti-proliferative agent which has been found to reduce smooth muscle cell proliferation and migration. It has been found that stents coated with such bioactive materials are beneficial in preventing restenosis after implantation of a stent or similar device in a patient. Preferably, the Paclitaxel present on the surface of the stent, after evaporation of the carrier solution, is at least 80% amorphous. In certain circumstances the amorphous form of the drug may be more effective than the crystalline form. The percentage of amorphous drug can be determined by removal of the drug from the surface. Firstly, the surface is dipped in a media which removes only the amorphous form of the drug from the surface, and secondly the surface is dipped in a solvent which removes all forms of the drug from the surface. A simple calculation is then performed to determine the percentage of the total drug present provided by the amorphous form.

Various embodiments of the invention are described with reference to the arrangement of the device shown in figure 1. The implantable medical device 10 of the present invention may be at least one of or any portion of a stent, a catheter, a guidewire, a cannula, a stent a vascular or other graft, a cardiac pacemaker lead or lead tip, a cardiac defibrillator lead or lead tip, a heart valve, or an orthopedic device, appliance, implant, or replacement. Such devices are useful in a variety of locations in a patient, such as in the oesophagus, trachea, colon, biliary tract, urinary tract and vascular system.

The base material 12 should be suitable for the intended use of the device. Preferably, the base material is biocompatible. It may be biodegradable or non-biodegradable. The base material may include a radiopaque material for identifying the location of the device 10 by X-ray or fluoroscopy during or after its introduction into the patient.

The base material 12 may comprise a metal, an alloy or a polymer, for example. The base material may include at least one of stainless steel, tantalum, titanium, nitinol, gold, platinum, inconel, iridium, silver, tungsten, colbolt, chromium, or another biocompatible metal, or alloys of any of these; carbon or carbon fiber; cellulose acetate, cellulose nitrate, silicone, polyethylene teraphthalate, polyurethane, polyamide, polyester, polyorthoester, polyanhydride, polyether sulfone, polycarbonate, polypropylene, high molecular weight polyethylene, polytetrafluoroethylene, or another biocompatible polymeric material, or mixtures or copolymers of these; polylactic acid, polyglycolic acid or copolymers thereof, a polyanhydride, polycaprolactone, polyhydroxybutyrate valerate or another biodegradable polymer, or mixtures or copolymers of these; a protein, an extracellular matrix component, collagen, fibrin or another biologic agent; or a suitable mixture of any of these.

Nitinol is particularly useful as the base material 12 when the device is a stent.

The contact surface may substantially entirely comprise either polar or non-polar components, and others may comprise a mix of polar and non-polar components. In the case of a device comprising a metal or metal alloy base material 12, the contact surface may comprise an oxide layer comprising oxides of the metals of the base material. In fact the majority of the contact surface may comprise such oxides. The contact surface 14 may comprise the base material itself, and/or other compounds derived from the base material or from the atmosphere. Of course impurities, or any compounds derived from the impurities may also exist at the contact surface.

Where the medical device comprises a polymer bulk material, the contact surface may comprise an oxide of the polymer. The contact surface of a polymer may comprise polar and non-polar components, or may comprise only non-polar components.

Where the bioactive material is predominantly polar and the contact surface is modified to increase the polar component, the surface may be, for example, bombarded with oxygen ions to as to ionise components at the surface and therefore render them polar.

Whether the surface is modified to increase the polar or non-polar component, roughening the surface has the effect of increasing the surface area exposed for bonding and therefore has the effect of magnifying the polar or non-polar nature of the surface.

As mentioned hereinbefore, the bioactive material 16 may be any drug, medicament or material, or combination thereof which can be coated onto a contact surface. The bioactive material may be coated onto the surface by a number of known techniques including, for example, spraying, vaccum deposition, and rolling the surface in the bioactive material.

A vast range of bioactive materials are known and maybe used, for example heparin, covalent heparin, or another thrombin inhibitor, hirudin, hirulog, argatroban, D-phenylalanyl-L-poly-L-arginyl chloromethyl ketone, or another antithrombogenic agent, or mixtures thereof; urokinase, streptokinase, a tissue plasminogen activator, or another thrombolytic agent, or mixtures thereof; a fibrinolytic agent; a vasospasm inhibitor; a calcium channel blocker, a nitrate, nitric oxide, a nitric oxide promoter or another vasodilator; Hytrin(R) or other antihypertensive agents; an antimicrobial agent or antibiotic; aspirin, ticlopidine, a glycoprotein IIb/IIIa inhibitor or another inhibitor of surface glycoprotein receptors, or another antiplatelet agent; colchicine or another antimitotic, or another microtubule inhibitor, dimethyl sulfoxide (DMSO), a retinoid or another antisecretory agent; cytochalasin or another actin inhibitor; or a remodelling inhibitor; deoxyribonucleic acid, an antisense nucleotide or another agent for molecular genetic intervention; methotrexate or another antimetabolite or antiproliferative agent; tamoxifen citrate, Taxol(R) or the derivatives thereof, or other anti-cancer chemotherapeutic agents; dexamethasone, dexamethasone sodium phosphate, dexamethasone acetate or another dexamethasone derivative, or another anti-inflammatory steroid or non-steroidal antiinflammatory agent; cyclosporin or another immunosuppressive agent; trapidal (a PDGF antagonist), angiopeptin (a growth hormone antagonist), angiogenin, a growth factor or an anti-growth factor antibody, or another growth factor antagonist; dopamine, bromocriptine mesylate, pergolide mesylate or another dopamine agonist; <60>Co(5.3 year half life), <192>Ir (73.8 days), <32>P(14.3 days), <111>In(68 hours), <90>Y(64 hours), <99m>Tc(6 hours) or another radiotherapeutic agent; iodine-containing compounds, barium-containing compounds, gold, tantalum, platinum, tungsten or another heavy metal functioning as a radiopaque agent; a peptide, a protein, an enzyme, an extracellular matrix component, a cellular component or another biologic agent; captopril, enalapril or another angiotensin converting enzyme (ACE) inhibitor; ascorbic acid, alpha tocopherol, superoxide dismutase, deferoxamine, a 21-aminosteroid (lasaroid) or another free radical scavenger, iron chelator or antioxidant; a <14>C-, <3>H-, <131>I-, <32>P- or <36>S-radiolabelled form or other radiolabelled form of any of the foregoing; estrogen or another sex hormone; AZT or other antipolymerases; acyclovir, famciclovir, rimantadine hydrochloride, ganciclovir sodium, Norvir, Crixivan, or other antiviral agents; 5-aminolevulinic acid, meta-tetrahydroxyphenylchlorin, hexadecafluoro zinc phthalocyanine, tetramethyl hematoporphyrin, rhodamine 123 or other photodynamic therapy agents; an IgG2 Kappa antibody against Pseudomonas aeruginosa exotoxin A and reactive with A431 epidermoid carcinoma cells, monoclonal antibody against the noradrenergic enzyme dopamine beta-hydroxylase conjugated to saporin or other antibody targeted therapy agents; gene therapy agents; and enalapril and other prodrugs; Proscar(R), Hytrin(R) or other agents for treating benign prostatic hyperplasia (BHP) or a mixture of any of these; and various forms of small intestine submucosa (SIS). No coating is required over the bioactive material to control the elution rate of the bioactive material from the medical device.

The bioactive material 16 may be coated on one surface of the device 10. Second and further surfaces of the device may be coated with second and further bioactive materials, and others may have no bioactive material coated thereon. The contact surfaces with no bioactive material coated thereon may be left bare, or may be coated with other materials, depending on the application of the device. The second and further contact surfaces of the device may have a proportion of polar and non-polar components different from the proportion of polar and non-polar components of the at least one contact surface. Each surface may be modified to optimise bonding with the particular bioactive material which is to be coated thereon. The different bioactive materials can be coated on different regions of the device so as to target delivery of the materials into specific regions of the body.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. An implantable medical device (10) including at least one contact surface (14) having a bioactive material (16) coated directly thereon, wherein:
the at least one contact surface (14) has a defined surface energy density comprising a polar component and a non-polar component; the at least one contact surface is a modified contact surface having adjusted proportions of polar and/or non-polar component;
the bioactive material (16) has a defined surface energy density comprising a polar component and a non-polar component; and
wherein the polar component of the surface energy of the at least one contact surface (14) is matched to within +/- 15 mN/m of the polar component of the surface energy of the bioactive material (16) and the non-polar component of the surface energy of the at least one contact surface (14) is matched to within +/-15 mN/m of the non-polar component of the surface energy of the bioactive material (16).

2. An implantable medical device according to claim 1 wherein at least one of:
the polar components of the surface energies of the at least one contact surface (14) and of the bioactive material (16) are matched to within +/- 10 mN/m and the non-polar components of the surface energies of the at least one contact surface (14) and of the bioactive material (16) are matched to within +/- 10 mN/m; and
the polar components of the surface energies of the at least one contact surface (14) and of the bioactive material (16) are matched to within +/- 5 mN/m and the non-polar components of the surface energies of the at least one contact surface (14) and of the bioactive material (16) are matched to within +/- 5 mN/m.

3. An implantable medical device according to any preceding claim wherein the medical device (16) includes a structure comprising a base material (12), and wherein the at least one contact surface (14) is a surface of the base material (12), the contact surface (14) comprising at least one of non-polar and polar components.

4. An implantable medical device according to claim 3 wherein the bioactive material (16) is predominantly polar or non-polar.

5. An implantable medical device according to any preceding claim wherein at least one contact surface (14) is a modified surface, wherein the contact surface (14) comprises a given proportion of non-polar to polar components and wherein the bioactive material (16) comprises a given proportion of non-polar to polar components, the at least one contact surface (14) having been modified to alter the proportion of polar and non-polar components to be closer to the proportion of polar and non-polar components in the bioactive material (16).

6. An implantable medical device according to any preceding claim wherein the surface energy density of the at least one contact surface (14) is at least one of:
at least 40 mN/m;
between 40 and 60 mN/m; and
between 50 and 60 mN/m.

7. An implantable medical device according to any preceding claim, wherein the at least one contact surface (14) is at least one of:
a selectively etched surfaced, the surface having been selectively etched to remove at least some of one of the polar and non-polar components at the at least one contact surface;
a selectively polished surface, the surface having been selectively polished to remove at least some of one of the polar and non-polar components at the at least one contact surface; and
an at least partially passivated or ionised surface, the surface having been least partially passivated or ionised to alter the proportion of polar to non-polar components at the at least one contact surface.

8. An implantable medical device according to any of claims 3 to 7, wherein the base material (12) is selectively doped so as to modify the at least one contact surface, said doping altering the proportion of polar to non-polar components at the at least one contact surface.

9. An implantable medical device according to any preceding claim, wherein the medical device (10) comprises a nickel titanium alloy.

10. An implantable medical device according to claim 9, wherein the at least one contact surface (14) comprises nickel and wherein the at least one contact surface is chemically treated to reduce the amount of nickel at said surface.

11. An implantable medical device according to claim 10, wherein the at least one contact surface (14) comprises at least one of:
no more than 5% nickel;
no more than 3% nickel; and
no more than 2.5% nickel.

12. A method of making an implantable medical device including a structure comprising a base material (12), the structure including at least one contact surface (14) of the base material, the contact surface (14) comprising at least one of non-polar and polar components; and a bioactive material (16) coating disposed directly on the at least one contact surface, the bioactive material (16) being predominantly polar or non-polar; the method including the steps of:
modifying the at least one contact surface (14) to alter the proportion of polar to non-polar components to be closer to the proportion of polar to non-polar components in the bioactive material (16);
matching the polar component of the surface energy of the at least one contact surface (14) to within +/- 15 mN/m of the polar component of the surface energy of the bioactive material and matching the non-polar component of the surface energy of the at least one contact surface to within +/- 15 mN/m of the non-polar component of the surface energy of the bioactive material; and
applying the bioactive material (16) directly to the at least one contact surface;
wherein a surface energy of the at least one contact surface (14) is above 40 mN/m.

13. A method according to claim 12, including the step of treating the at least one contact surface (14) prior to application of the bioactive material (16) so as to limit the surface energy of the at least one contact surface to between 40 and 60 mN/m.

14. A method according to any one of claims 12 and 13, wherein the modification step includes chemically modifying at least a part of the at least one contact surface (14) by at least one of:
selectively etching the at least one contact surface (14) to remove at least some of one of the polar and non-polar components at the at least one contact surface;
selectively polishing the at least one contact surface (14) to remove at least some of one of the polar and non-polar components at the at least one contact surface; and
at least partially selectively passivating or at least partially selectively ionising the at least one contact surface to alter the proportion of polar to non-polar components at the at least one contact surface; and
selectively doping the base material (12) so as to alter the proportion of polar to non-polar components at the at least one contact surface.

## Patentansprüche

1. Implantierbare medizinische Vorrichtung (10), die mindestens eine Kontaktoberfläche (14) einschließt, auf die direkt ein bioaktives Material (16) als Beschichtung aufgebracht ist, wobei:
die mindestens eine Kontaktoberfläche (14) eine definierte Oberflächenenergiedichte aufweist, die eine polare Komponente und eine unpolare Komponente umfasst; wobei die mindestens eine Kontaktoberfläche eine modifizierte Kontaktoberfläche mit angepassten Verhältnissen von polarer und/oder unpolarer Komponente ist;
das bioaktive Material (16) eine definierte Oberflächenenergiedichte aufweist, die eine polare Komponente und eine unpolare Komponente umfasst; und
wobei die polare Komponente der Oberflächenenergie der mindestens einen Kontaktoberfläche (14) auf innerhalb +/-15 mN/m zu der polaren Komponente der Oberflächenenergie des bioaktiven Materials (16) angeglichen ist, und die unpolare Komponente der Oberflächenenergie der mindestens einen Kontaktoberfläche (14) auf innerhalb +/- 15 mN/m zu der unpolaren Komponente der Oberflächenenergie des bioaktiven Materials (16) angeglichen ist.

2. Implantierbare medizinische Vorrichtung nach Anspruch 1, wobei mindestens eines der folgenden zutrifft:
die polaren Komponenten der Oberflächenenergien von der mindestens einen Kontaktoberfläche (14) und dem bioaktiven Material (16) sind auf innerhalb von +/-10 mN/m angeglichen und die unpolaren Komponenten der Oberflächenenergien von der mindestens einen Kontaktoberfläche (14) und dem bioaktiven Material (16) sind auf innerhalb +/-10 mN/m angeglichen; und
die polaren Komponenten der Oberflächenenergien von der mindestens einen Kontaktoberfläche (14) und dem bioaktiven Material (16) sind auf innerhalb von +/- 5 mN/m angeglichen und die unpolaren Komponenten der Oberflächenenergien von der mindestens einen Kontaktoberfläche (14) und dem bioaktiven Material (16) sind auf innerhalb +/- 5 mN/m angeglichen.

3. Implantierbare medizinische Vorrichtung nach einem der vorhergehenden Ansprüche, wobei die medizinische Vorrichtung (16) eine Struktur einschließt, die ein Basismaterial (12) einschließt, und wobei die mindestens eine Kontaktoberfläche (14) eine Oberfläche des Basismaterials (12) ist, wobei die Kontaktoberfläche (14) mindestens eine der unpolaren und polaren Komponenten umfasst.

4. Implantierbare medizinische Vorrichtung nach Anspruch 3, wobei das bioaktive Material (16) vorwiegend polar oder unpolar ist.

5. Implantierbare medizinische Vorrichtung nach einem der vorhergehenden Ansprüche, wobei mindestens eine Kontaktoberfläche (14) eine modifizierte Oberfläche ist, wobei die Kontaktoberfläche (14) ein gegebenes Verhältnis von unpolaren zu polaren Komponenten umfasst, und wobei das bioaktive Material (16) ein gegebenes Verhältnis von unpolaren zu polaren Komponenten umfasst, wobei die mindestens eine Kontaktoberfläche (14) modifiziert worden ist, um das Verhältnis von polaren zu unpolaren Komponenten zu ändern, so dass es näher an dem Verhältnis von polaren und unpolaren Komponenten in dem bioaktiven Material (16) ist.

6. Implantierbare medizinische Vorrichtung nach einem der vorhergehenden Ansprüche, wobei die Oberflächenenergiedichte der mindestens einen Kontaktoberfläche (14) mindestens eine der folgenden ist:
mindestens 40 mN/m;
zwischen 40 und 60 mN/m; und
zwischen 50 und 60 mN/m.

7. Implantierbare medizinische Vorrichtung nach einem der vorhergehenden Ansprüche, wobei die mindestens eine Kontaktoberfläche (14) mindestens eine der folgenden ist:
eine selektiv geätzte Oberfläche, wobei die Oberfläche selektiv geätzt worden ist, um mindestens etwas von einer der polaren und unpolaren Komponenten an der mindestens einen Kontaktoberfläche zu entfernen;
eine selektiv polierte Oberfläche, wobei die Oberfläche selektiv poliert worden ist, um mindestens etwas von einer der polaren und unpolaren Komponenten an der mindestens einen Kontaktoberfläche zu entfernen; und
eine mindestens teilweise passivierte oder ionisierte Oberfläche, wobei die Oberfläche mindestens teilweise passiviert oder ionisiert worden ist, um das Verhältnis von polaren zu unpolaren Komponenten an der mindestens einen Kontaktoberfläche zu ändern.

8. Implantierbare medizinische Vorrichtung nach einem der Ansprüche 3 bis 7, wobei das Basismaterial (12) selektiv dotiert ist, um so die mindestens eine Kontaktoberfläche zu modifizieren, wobei das Dotieren das Verhältnis der polaren zu unpolaren Komponenten der mindestens einen Kontaktoberfläche ändert.

9. Implantierbare medizinische Vorrichtung nach einem der vorhergehenden Ansprüche, wobei die medizinische Vorrichtung (10) eine Nickel-TitanLegierung umfasst.

10. Implantierbare medizinische Vorrichtung nach Anspruch 9, wobei die mindestens eine Kontaktoberfläche (14) Nickel umfasst, und wobei die mindestens eine Kontaktoberfläche chemisch behandelt wird, um die Menge an Nickel an der Oberfläche zu reduzieren.

11. Implantierbare medizinische Vorrichtung nach Anspruch 10, wobei die mindestens eine Kontaktoberfläche (14) mindestens eines der folgenden umfasst:
nicht mehr als 5 % Nickel;
nicht mehr als 3 % Nickel; und
nicht mehr als 2,5 % Nickel.

12. Verfahren zur Fertigung einer implantierbaren medizinischen Vorrichtung, die eine Struktur einschließt, die ein Basismaterial (12) umfasst, wobei die Struktur mindestens eine Kontaktoberfläche (14) des Basismaterials einschließt, wobei die Kontaktoberfläche (14) mindestens eine von unpolaren und polaren Komponenten umfasst; und wobei eine Beschichtung aus bioaktivem Material (16) direkt auf der mindestens einen Kontaktoberfläche angeordnet ist, wobei das bioaktive Material (16) vorwiegend polar oder unpolar ist; wobei das Verfahren die Schritte einschließt:
Modifizieren der mindestens einen Kontaktoberfläche (14), um das Verhältnis von polaren zu unpolaren Komponenten zu ändern, so dass es näher an dem Verhältnis von polaren zu unpolaren Komponenten in dem bioaktiven Material (16) liegt;
Angleichen der polaren Komponente der Oberflächenenergie der mindestens einen Kontaktoberfläche (14) auf innerhalb +/- 15 mN/m zu der polaren Komponente der Oberflächenenergie von dem bioaktiven Material, und Angleichen der unpolaren Komponente der Oberflächenenergie der mindestens einen Kontaktoberfläche auf innerhalb +/- 15 mN/m zu der unpolaren Komponente der Oberflächenenergie von dem bioaktiven Material; und
Aufbringen des bioaktiven Materials (16) direkt auf die mindestens eine Kontaktoberfläche;
wobei eine Oberflächenenergie der mindestens einen Kontaktoberfläche (14) über 40 mN/m beträgt.

13. Verfahren nach Anspruch 12, das den Schritt des Behandelns der mindestens einen Kontaktoberfläche (14) vor der Aufbringung des bioaktiven Materials (16) einschließt, um so die Oberflächenenergie der mindestens einen Kontaktoberfläche auf zwischen 40 und 60 mN/m zu begrenzen.

14. Verfahren nach einem der Ansprüche 12 und 13, wobei der Modifikationsschritt chemisches Modifizieren von mindestens einem Teil der mindestens einen Kontaktoberfläche (14) durch mindestens eines der folgenden einschließt:
selektives Ätzen der mindestens einen Kontaktoberfläche (14), um mindestens etwas von einer der polaren und unpolaren Komponenten an der mindestens einen Kontaktoberfläche zu entfernen;
selektives Polieren der mindestens einen Kontaktoberfläche (14), um mindestens etwas von einer der polaren und unpolaren Komponenten an der mindestens einen Kontaktoberfläche zu entfernen; und
mindestens teilweise selektives Passivieren oder mindestens teilweise selektives Ionisieren der mindestens einen Kontaktoberfläche, um das Verhältnis der polaren zu unpolaren Komponenten an der mindestens einen Kontaktoberfläche zu ändern; und
selektives Dotieren des Basismaterials (12), um so das Verhältnis von polaren zu unpolaren Komponenten an der mindestens einen Kontaktoberfläche zu ändern.

## Revendications

1. Dispositif médical implantable (10) comprenant au moins une surface de contact (14) sur laquelle est directement revêtu un matériau bioactif (16), dans lequel :
l'au moins une surface de contact (14) a une densité d'énergie de surface définie comprenant une composante polaire et une composante non polaire ; l'au moins une surface de contact est une surface de contact modifiée ayant des proportions ajustées de composante polaire et/ou non polaire ;
le matériau bioactif (16) a une densité d'énergie de surface définie comprenant une composante polaire et une composante non polaire ; et
dans lequel la composante polaire de l'énergie de surface de l'au moins une surface de contact (14) correspond à +/-15 mN/m de la composante polaire de l'énergie de surface du matériau bioactif (16) et la composante non polaire de l'énergie de surface de l'au moins une surface de contact (14) correspond à +/-15 mN/m de la composante non polaire de l'énergie de surface du matériau bioactif (16).

2. Dispositif médical implantable selon la revendication 1, dans lequel au moins un parmi :
les composantes polaires des énergies de surface de l'au moins une surface de contact (14) et du matériau bioactif (16) correspondent à +/-10 mN/m et les composantes non polaires des énergies de surface de l'au moins une surface de contact (14) et du matériau bioactif (16) correspondent à +/-10 mN/m ; et
les composantes polaires des énergies de surface de l'au moins une surface de contact (14) et du matériau bioactif (16) correspondent à +/-5 mN/m et les composantes non polaires des énergies de surface de l'au moins une surface de contact (14) et du matériau bioactif (16) correspondent à +/-5 mN/m.

3. Dispositif médical implantable selon l'une quelconque des revendications précédentes, le dispositif médical (16) comprenant une structure comprenant un matériau de base (12), et dans lequel l'au moins une surface de contact (14) est une surface du matériau de base (12), la surface de contact (14) comprenant au moins une des composantes non polaires et polaires.

4. Dispositif médical implantable selon la revendication 3, dans lequel le matériau bioactif (16) est principalement polaire ou non polaire.

5. Dispositif médical implantable selon l'une quelconque des revendications précédentes, dans lequel au moins une surface de contact (14) est une surface modifiée, dans lequel la surface de contact (14) comprend une proportion donnée des composantes non polaires aux composantes polaires et dans lequel le matériau bioactif (16) comprend une proportion donnée des composantes non polaires aux composantes polaires, l'au moins une surface de contact (14) ayant été modifiée pour changer la proportion de composantes polaires et non polaires pour qu'elle soit plus proche de la proportion de composantes polaires et non polaires dans le matériau bioactif (16).

6. Dispositif médical implantable selon l'une quelconque des revendications précédentes, dans lequel la densité d'énergie de surface de l'au moins une surface de contact (14) est : au moins 40 mN/m ; et/ou
entre 40 et 60 mN/m ; et/ou
entre 50 et 60 mN/m.

7. Dispositif médical implantable selon l'une quelconque des revendications précédentes, dans lequel l'au moins une surface de contact (14) est :
une surface gravée sélectivement, la surface ayant été sélectivement gravée pour éliminer au moins une partie des composantes polaires et non polaires au niveau de l'au moins une surface de contact ; et/ou
une surface polie sélectivement, la surface ayant été sélectivement polie pour éliminer au moins une partie des composantes polaires et non polaires au niveau de l'au moins une surface de contact ; et/ou
une surface au moins partiellement passivée ou ionisée, la surface ayant été au moins partiellement passivée ou ionisée pour changer la proportion des composantes polaires aux composantes non polaires au niveau de l'au moins une surface de contact.

8. Dispositif médical implantable selon l'une quelconque des revendications 3 à 7, dans lequel le matériau de base (12) est sélectivement dopé de façon à modifier l'au moins une surface de contact, ledit dopage changeant la proportion des composantes polaires aux composantes non polaires au niveau de l'au moins une surface de contact.

9. Dispositif médical implantable selon l'une quelconque des revendications précédentes, le dispositif médical (10) comprenant un alliage de nickel et de titane.

10. Dispositif médical implantable selon la revendication 9, dans lequel l'au moins une surface de contact (14) comprend du nickel et dans lequel l'au moins une surface de contact est chimiquement traitée pour réduire la quantité de nickel au niveau de ladite surface.

11. Dispositif médical implantable selon la revendication 10, dans lequel l'au moins une surface de contact (14) comprend :
5 % de nickel ou moins ; et/ou
3 % de nickel ou moins ; et/ou
2,5 % de nickel ou moins.

12. Procédé de fabrication d'un dispositif médical implantable comprenant une structure comprenant un matériau de base (12), la structure comprenant au moins une surface de contact (14) du matériau de base, la surface de contact (14) comprenant au moins une des composantes non polaires et polaires ; et un revêtement de matériau bioactif (16) disposé directement sur l'au moins une surface de contact, le matériau bioactif (16) étant principalement polaire ou non polaire ; le procédé comprenant les étapes consistant à :
modifier l'au moins une surface de contact (14) pour changer la proportion des composantes polaires aux composantes non polaires pour qu'elle soit plus proche de la proportion des composantes polaires aux composantes non polaires dans le matériau bioactif (16) ;
faire correspondre la composante polaire de l'énergie de surface de l'au moins une surface de contact (14) à +/-15 mN/m de la composante polaire de l'énergie de surface du matériau bioactif et faire correspondre la composante non polaire de l'énergie de surface de l'au moins une surface de contact à +/-15 mN/m de la composante non polaire de l'énergie de surface du matériau bioactif ; et
appliquer le matériau bioactif (16) directement sur l'au moins une surface de contact ;
dans lequel une énergie de surface de l'au moins une surface de contact (14) est supérieure à 40 mN/m.

13. Procédé selon la revendication 12, comprenant les étapes consistant à traiter l'au moins une surface de contact (14) avant l'application du matériau bioactif (16) de façon à limiter l'énergie de surface de l'au moins une surface de contact entre 40 et 60 mN/m.

14. Procédé selon l'une quelconque des revendications 12 et 13, dans lequel l'étape de modification consiste à modifier chimiquement au moins une partie de l'au moins une surface de contact (14) par :
la gravure sélective de l'au moins une surface de contact (14) pour éliminer au moins une partie des composantes polaires et non polaires au niveau de l'au moins une surface de contact ; et/ou
le polissage sélectif de l'au moins une surface de contact (14) pour éliminer au moins une partie des composantes polaires et non polaires au niveau de l'au moins une surface de contact ; et/ou
la passivation au moins partiellement sélective ou l'ionisation au moins partiellement sélective de l'au moins une surface de contact pour changer la proportion des composantes polaires aux composantes non polaires au niveau de l'au moins une surface de contact ; et/ou
le dopage sélectif du matériau de base (12) de façon à modifier la proportion des composantes polaires aux composantes non polaires au niveau de l'au moins une surface de contact.
